# EUROPÄISCHE PATENTSCHRIFT

(11) **EP 0 891 226 B1**
(45) Veröffentlichungstag und Bekanntmachung des Hinweises auf die Patenterteilung: **10.11.1999**
(21) Anmeldenummer: 97914254.4
(22) Anmeldetag: 17.03.1997
(51) Int. Cl.: B01J 23/58, C07C 67/055, C07C 69/01

(54) **KATALYSATOR UND VERFAHREN ZUR HERSTELLUNG VON VINYLACETAT**
CATALYST AND PROCESS FOR THE PREPARATION OF VINYL ACETATE
CATALYSEUR ET PROCEDE PERMETTANT DE PRODUIRE DE L'ACETATE DE VINYLE

(30) Priorität: 04.04.1996 DE 19613791
(43) Veröffentlichungstag der Anmeldung: 20.01.1999
(73) Patentinhaber: Celanese GmbH, 60439 Frankfurt (DE)
(72) Erfinder: ABEL, Roland, Corpus Christi, TX 78418 (US); NICOLAU, Ioan, Corpus Christi, TX 78413 (US); HOPF, Erich, D-63584 Gründau (DE); KIEMEL, Rainer, D-63526 Erlensee (DE)
(86) Internationale Anmeldenummer: EP9701327
(87) Internationale Veröffentlichungsnummer: WO9737759

(56) Entgegenhaltungen:
- EP-A- 0 672 453
- EP-A- 0 685 451
- US-A- 3 743 607
- US-A- 3 939 199
- US-A- 4 048 096
- US-A- 5 332 710

## Beschreibung

Die vorliegende Erfindung betrifft einen Katalysator, der Palladium, Gold und eine Alkalimetallverbindung enthält, ein Verfahren zu seiner Herstellung und seine Verwendung zur Herstellung von Vinylacetat aus Essigsäure, Ethylen und Sauerstoff oder Sauerstoff enthaltenden Gasen.

Aus dem Stand der Technik ist bekannt, daß Vinylacetat aus Ethylen, Sauerstoff und Essigsäure durch Reaktion an Katalysatoren hergestellt werden kann, die auf einem Trägermaterial (wie etwa Siliziumdioxid) Palladium, Gold und eine Alkalimetallverbindung enthalten. Solche Katalysatoren zeigen eine gute Aktivität und bilden im allgemeinen wenig Kohlendioxid und Ethylacetat. Obwohl Hochsieder als weitere Nebenprodukte in gering erscheinenden Mengen gebildet werden, so stellen sie doch verfahrenstechnisch und ökologisch ein Problem dar. Als Hochsieder bezeichnet man dabei insbesondere die Verbindungen Ethylidendiacetat, Ethylenglykol, sowie Diacetoxyethylene.

Literaturstellen, die die Herstellung von derartigen Katalysatoren zur kommerziellen Produktion von Vinylacetat offenbaren, beschreiben im allgemeinen Verfahren zur Ablagerung der Edelmetalle in einer Schale auf dem Katalysatorträger.

US-A-4.048.096 beschreibt die Herstellung eines Katalysators zur Vinylacetatproduktion, indem die gelösten Edelmetallsalze vom Träger aus einer Lösung adsorbiert werden, die das gleiche Volumen wie die Poren des Trägermaterials hat, wobei die Trägerteilchen in einem rotierenden Gefäß bewegt werden. Die Salze werden dann mit Alkalien fixiert, ohne vorher den Träger zu trocknen.

US-A-5.332.710 offenbart die Herstellung eines Katalysators zur Vinylacetatproduktion, wobei man die unlöslichen Edelmetallsalze auf den Trägerteilchen niederschlägt, indem man diese während der Niederschlagung mittels Alkalien mindestens eine halbe Stunde in einer Trommel rotierend bewegt.

Überraschenderweise wurde nun gefunden, daß der Zusatz von Bor oder Borverbindungen die Selektivität des Katalysators verbessert, wobei insbesondere die Hochsiederbildung deutlich verringert wird. Hochsieder sind insbesondere die eingangs genannten Verbindungen.

Ein Gegenstand der Erfindung ist ein Katalysator zur Herstellung von Vinylacetat in der Gasphase aus Ethylen, Essigsäure und Sauerstoff oder Sauerstoff enthaltenden Gasen bei gleichzeitig geringer Hochsiederbildung, der Palladium und/oder dessen Verbindungen, Gold und/oder dessen Verbindungen sowie Alkalimetallverbindungen auf einem teilchenförmigen Träger enthält, dadurch gekennzeichnet, daß der Katalysator zusätzlich Bor oder Borverbindungen enthält.

Weitere Gegenstände der Erfindung sind ein Verfahren zur Herstellung des Katalysators mittels der in Anspruch 6 genannten Merkmale, sowie seine Verwendung in einem Verfahren zur Herstellung von Vinylacetat mittels der in Anspruch 14 genannten Merkmale.

Es handelt sich dabei zum einen um ein Verfahren zur Herstellung eines Katalysators zur Produktion von Vinylacetat in der Gasphase aus Ethylen, Essigsäure und Sauerstoff oder Sauerstoff enthaltenden Gasen bei gleich zeitig geringer Hochsiederbildung, der Palladium und/oder dessen Verbindungen, Gold und/oder dessen Verbindungen sowie Alkalimetallverbindungen auf einem teilchenförmigen Träger enthält, wobei der Katalysator hergestellt wird indem man
a) den Träger mit löslichen Palladium- und Goldverbindungen imprägniert;
b) die löslichen Palladium- und Goldverbindungen durch eine alkalisch reagierende Lösung auf dem Träger in unlösliche Verbindungen überführt;
c) die unlöslichen Palladium- und Goldverbindungen auf dem Träger durch ein Reduktionsmittel in flüssiger Phase reduziert;
d) den Träger wäscht und anschließend trocknet;
e) den Träger mit einer löslichen Alkalimetallverbindung imprägniert; und
f) den Träger abschließend bei höchstens 150°C trocknet,
   dadurch gekennzeichnet, daß vor der abschließenden Trocknung Bor oder Borverbindungen auf den Katalysator aufgebracht werden.

Zum anderen handelt es sich um ein Verfahren zur Herstellung von Vinylacetat in der Gasphase aus Ethylen, Essigsäure und Sauerstoff oder Sauerstoff enthaltenden Gasen bei gleichzeitig geringer Hochsiederbildung an dem erfindungsgemäßen Katalysator.

Der Gehalt des Katalysators an Bor beträgt bevorzugt 0,01 bis 1 Gew.-%, insbesondere 0,01 bis 0,2 Gew.-%. Das Bor wird in Form seiner Verbindungen, bevorzugt in Form von Boraten, auf den Träger aufgebracht. Das Aufbringen kann im oben genannten Schritt a) zusammen mit den löslichen Palladium- und Goldverbindungen, in Schritt b) zusammen mit der alkalischen Lösung, oder unter Verwendung einer Boratlösung als alkalische Lösung, im Schritt e) mit der löslichen Alkaliverbindung oder in einem eigenen Schritt vor der letzten Trocknung des Trägers erfolgen. Bevorzugt ist das Aufbringen in Schritt b).

Die Trägerteilchen des erfindungsgemäßen Katalysators können eine beliebige geometrische Form haben, beispielsweise die Form von Kugeln, Tabletten oder Zylindern in regelmäßiger oder unregelmäßiger Ausführung. Die Abmessung der Trägerteilchen liegt im allgemeinen zwischen 1 und 8 mm. Bevorzugt ist die Kugelform, zum Beispiel Kugeln mit einem Durchmesser von 4 bis 8 mm. Die Trägerteilchen werden im allgemeinen Pellets genannt.

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Träger dieser Art mit einer spezifischen Oberfläche von 50 bis 300 m²/g (gemessen nach der BET-Methode) und einem mittleren Porenradius von 50 bis 2000 Å (gemessen mit Quecksilber-Porosimetrie), vor allem Kieselsäure (SiO₂) und SiO₂-Al₂O₃-Gemische.

Vorzugsweise liegt das Gesamtporenvolumen des Trägers bei 0,4 bis 1,2 ml/g, wobei weniger als 10 % dieses Volumens von "Mikroporen" mit einem Porendurchmesser unter 30 Å (Angström) gebildet werden sollten. Solche Träger können aus aerogenem SiO₂ oder einem aerogenen SiO₂-Al₂O₃-Gemisch hergestellt werden, welches in Form von glasigen Mikrokugeln vorliegt, die beispielsweise durch Flammenhydrolyse von Siliciumtetrachlorid oder eines Siliciumtetrachlorid-Aluminiumtrichlorid-Gemisches in einer Knallgasflamme hergestellt werden können (US-A-3 939 199). Im Handel sind diese Mikrokugeln unter dem Namen ®Aerosil oder ®Cabosil erhältlich.

Die gelösten Gold- und Palladiumsalze werden in den Poren des Trägermaterials adsorbiert, was im Stand der Technik als die Porenvolumenimprägniermethode bezeichnet wird. Die so imprägnierten Träger werden mit einer alkalischen Lösung behandelt, wobei die Verwendung einer Boratlösung bevorzugt ist, um die Edelmetalle als unlösliche Verbindungen abzuscheiden. Diese Verbindungen werden anschließend einer reduktiven Behandlung unterzogen, wobei das Reduktionsmittel in flüssiger Phase vorliegt.

Als Lösungsmittel für die katalytisch aktiven Substanzen sind vor allem Wasser oder unsubstituierte Carbonsäuren mit 2 bis 10 Kohlenstoffatomen, wie Essigsäure, Propionsäure, n- und iso-Buttersäure und die verschiedenen Valeriansäuren geeignet. Wegen ihrer physikalischen Eigenschaften und auch aus wirtschaftlichen Gründen wird als Carbonsäure vorzugsweise Essigsäure eingesetzt. Die zusätzliche Verwendung eines inerten Lösungsmittels ist dann zweckmäßig, wenn eine Carbonsäure eingesetzt wird, in der die Substanzen nicht ausreichend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit der Carbonsäure mischbar sind, zum Beispiel Wasser, oder Ether, wie Tetrahydrofuran oder Dioxan, aber auch Kohlenwasserstoffe, wie Benzol.

Im folgenden werden zwei geeignete, mit I und II bezeichnete Methoden für die Herstellung des erfindungsgemäßen Katalysators beschrieben. Ein bestimmter Schritt von Methode II kann in 2 Varianten A) und B) ausgeführt werden.

Nach Methode I werden lösliche Gold- und Palladiumsalze in einer solchen Menge Lösungsmittel gelöst, daß die Lösung etwa 90 - 110% des Porenvolumens des Trägermaterials einnimmt. Palladium-(II)-chlorid, Natrium-Palladium-(II)-chlorid und Palladium-(II)-nitrat sind Beispiele geeigneter löslicher Palladiumverbindungen, und Gold-(III)-chlorid, Tetrachlorogold-(III)-säure sowie deren Alkalimetallsalze können als lösliche Goldverbindungen verwendet werden. Im allgemeinen werden von diesen Verbindungen solche Mengen benutzt, daß der fertige Katalysator zwischen etwa 2 und etwa 14 g/l, bevorzugt zwischen 4 und 8 g/l, Palladium, sowie zwischen etwa 1 und etwa 8 g/l, bevorzugt zwischen 2 und 5 g/l, Gold enthält. Dementsprechend beträgt der Goldanteil des Katalysators im allgemeinen etwa 10 bis 70 % der in ihm vorhandenen Palladiummasse.

Die Lösung wird im Träger adsorbiert und die Metalle werden fixiert, indem der Träger ausreichend lange in eine alkalische Lösung gegeben wird, die eine hinreichende Konzentration hat, um die unlöslichen Metallsalze auszufällen. Der Fixierschritt b) kann ausgeführt werden, indem die imprägnierten Träger in genügend alkalische Fixierlösung, um sie gänzlich zu bedecken, untergetaucht werden, wobei die Trägerteilchen vorzugsweise bewegt werden, etwa wie in US-A-5.332.710 beschrieben ist. Auf diese Methode wird hiermit direkt Bezug genommen (incorporation by reference). Gemäß US-A-5.332.710 werden die imprägnierten Trägerteilchen in einer alkalischen Lösung untergetaucht und ab dem Beginn der Abscheidung der unlöslichen Edelmetallverbindungen rotierend bewegt. Diese Rotation der Trägerteilchen in der alkalischen Lösung sollte ab dem Beginn der Behandlung mindestens eine halbe Stunde dauern, vorzugsweise eine Stunde. Rotation und Untertauchen können bis zu 4 Stunden dauern. Die behandelten Trägerteilchen können danach ruhend in der Fixierlösung belassen werden um sicherzustellen, daß eine vollständige Ausfällung der Edelmetallverbindungen erfolgt. Als alkalische Fixierlösung kommt jede Lösung in Betracht, die in der Lage ist, Gold und Palladium auszufällen, bevorzugt werden Boratlösungen verwendet.

Jede Art von Rotation oder ähnlicher Behandlung, die die Trägerteilchen in Bewegung hält, kann benutzt werden, da die genaue Art und Weise nicht kritisch ist. Wichtig ist jedoch die Intensität der Bewegung. Diese soll ausreichen, die gesamte Oberfläche der imprägnierten Träger gleichmäßig mit der alkalischen Fixierlösung zu benetzen. Die Bewegung der Trägerteilchen darf nicht so stark sein, daß die unlöslichen Edelmetallverbindungen dadurch verloren gehen, daß sie von der Trägeroberfläche abgerieben werden. Vorzugsweise soll die Rotationsgeschwindigkeit 1 bis 20 Umdrehungen pro Minute betragen, sie kann aber auch größer sein, je nach Art des Trägermaterials und der Menge des abzuscheidenden Edelmetalls. Die Umdrehungszahl kann unterschiedlich gewählt werden, und hängt auch von dem verwendeten Apparat ab, der Größe und der Form der Träger, der Art des Trägers, der Metallimprägnierung usw., sollte aber etwa den oben genannten Umdrehungszahlen entsprechen.

Die Fixierlösung besteht aus einer alkalischen Lösung, bevorzugt einer wäßrigen Lösung, die Borverbindungen enthält. Insbesondere ist es bevorzugt, wäßrige Lösungen von Borax, Kaliumtetraborat, oder Mischungen aus Alkalilauge und Borsäure zu verwenden. Die alkalische Lösung kann Puffereigenschaften aufweisen.

Anschließend werden die Schritte c) bis f) durchgeführt.

Nach Methode II wird ein geeigneter Katalysatorträger zunächst mit einer Lösung, die lösliche Palladium- und Goldverbindungen enthält, imprägniert. Getrennte Lösungen von Palladium- und Goldverbindungen können auch nacheinander verwendet werden, doch muß dann ein Trockenschritt zwischengeschaltet werden. Für eine effektive Imprägnierung soll das Volumen der Imprägnierlösung 95 bis 100 % des Porenvolumens des Katalysatorträgers betragen, bevorzugt ist der Bereich von 98 - 99 %. Nach der Imprägnierung des Trägers mit den löslichen Palladium- und Goldverbindungen wird der imprägnierte Träger getrocknet, bevor die Palladium- und Goldverbindungen als unlösliche Verbindungen fixiert werden. Der Fixierschritt besteht aus mindestens zwei getrennten Stufen der Behandlung mit der alkalischen Fixierlösung. In jeder dieser Stufen ist die Menge des eingesetzten alkalischen Reagenzes höchstens gleich der Stoffmenge, die erforderlich ist, um mit der gesamten Menge der auf dem Träger befindlichen löslichen Edelmetallsalze zu reagieren. Diese Stoffmenge kann größer als die stöchiometrisch zur Reaktion notwendige Stoffmenge sein. Vorzugsweise ist die Menge des alkalischen Reagenzes, die in jeder Fixierstufe angewendet wird, weniger als die zur vollständigen Reaktion mit den löslichen Edelmetallsalzen erforderliche Menge. Die erste Fixierstufe wird durchgeführt, indem der imprägnierte und dann getrocknete Träger mit der alkalischen Fixierlösung in Kontakt gebracht wird. Das Volumen der Fixierlösung entspricht dem Poronvolumen des trockenen Trägermaterials. Die Menge der darin enthaltenen alkalischen Verbindung soll so bemessen sein, daß das molare Verhältnis von Alkalimetall aus der alkalischen Verbindung zu Anionen aus dem löslichen Metallsalz von 0,7:1 bis 2:1 ist, und das Volumen der Lösung soll dem Absorptionsvermögen des Trägers im trockenen Zustand entsprechen. Die alkalische Fixierlösung wird zum Aufsaugen auf die bewegten Trägerteilchen gegossen, und diese werden für bis zu 24 Stunden, vorzugsweise 2 bis 8 Stunden, stehen gelassen.

Die zweite Fixierstufe kann in 2 Varianten A) und B) durchgeführt werden.

Variante A) wird derart durchgeführt, daß die ungetrockneten Trägerteilchen einer Zweiten Fixierlösung ausgesetzt werden. In dieser Lösung beträgt das molare Verhältnis zwischen Alkalimetall aus der alkalischen Verbindung zu Anion aus dem Metallsalz von etwa 0,2:1 bis 2:1. Das Lösungsvolumen soll die Träger mindestens gerade bedecken. Die Behandlung der Trägerteilchen mit der zweiten Fixierlösung soll bis zu 16 Stunden dauern, mindestens 2 Stunden, vorzugsweise mindestens 4 Stunden.

Nach Variante B) werden die Träger mit dem rotation- immersion-Verfahren nach U.S. Pat. 5.332.710 behandelt. In diesem Verfahren werden die bereits in der ersten Stufe fixierten Träger in der alkalischen Fixierlösung der zweiten Stufe untergetaucht, und darin während der Anfangsphase der zweiten Stufe rotierend bewegt. Diese Rotation sollte mindestens eine halbe Stunde dauern, bevorzugt ist eine Stunde. Die Behandlung kann bis zu 4 Stunden dauern, bevor die Träger in der Fixierlösung stehen gelassen werden, um eine vollständige Abscheidung zu gewährleisten. Auch hier kann jede Art von Apparat für die Rotation der Trägerteilchen benutzt werden. Wichtig ist das Ausmaß der Rotation. Diese muß ausreichen, alle Oberflächen der Trägerteilchen gleichmäßig mit der alkalischen Fixierlösung in Kontakt zu bringen. Sie darf nicht so stark sein, daß ein Abrieb der unlöslichen Metallverbindungen von der Trägeroberfläche einsetzt. Vorzugsweise soll die Umdrehungsgeschwindigkeit 1 bis 20 Umdrehungen pro Minute betragen, gegebenenfalls größer sein, je nach Art des Trägermaterials und der Menge Metall, die auf dem Träger niedergeschlagen werden soll. Die Umdrehungszahl hängt auch von der Art des benutzten Apparates, von der Größe und Form der Träger, von der Art der Träger, von der Metallbeaufschlagung etc. ab, sollte aber ungefähr an der oben genannten Umdrehungszahl orientiert werden.

Die Behandlung in der zweiten Fixierstufe kann der in der ersten Stufe äquivalent sein, indem eine Fixierlösung der gleichen Konzentration verwendet wird. Vorzugsweise soll das molare Gesamtverhältnis von Alkalimetall zu Anion aus dem Metallsalz für beide Fixierstufen zusammen zwischen 1,1:1 bis 3,3:1 betragen. Bevorzugt ist die Verwendung einer Boratlösung als Fixierlösung in beiden Stufen.

Anschließend an den Fixierschritt von Methode I oder den letzten Fixierschritt von Methode II werden die Träger mit einem Reduktionsmittel behandelt, um die ausgefällten Edelmetallsalze und -verbindungen, die sich darauf befinden, in die metallische Form zu überführen. Diese Reduktion wird in der flüssigen Phase ausgeführt, zum Beispiel mit wäßrigem Hydrazinhydrat oder einem Alkaliborhydrid, bevorzugt ist Natriumborhydrid. Vorzugsweise wird die Reduktion bei Normaltemperatur durchgeführt. Das Reduktionsmittel wird im Überschuß zugegeben, um sicherzustellen, daß die Gesamtheit der Metallsalze und -verbindungen in die metallische Form überführt werden. Auch die Borverbindungen können gegebenenfalls, abhängig von der Art des Reduktionsmittels, in elementares Bor überführt werden.

Die Trägerteilchen werden gewaschen, vorzugsweise mit destilliertem Wasser, um etwaige noch im Trägermaterial befindlichen Chloride zu entfernen, die vom Imprägnierschritt herrühren, und durch die Abscheidung der Edelmetalle freigesetzt wurden. Die Wäsche wird fortgesetzt, bis die Chloride vom Träger entfernt sind. Es sollen nicht mehr als 1000 ppm Chloride auf dem Katalysator verbleiben. Um den Erfolg des Waschvorganges sicherzustellen, kann die Waschflüssigkeit mit Silbernitratlösung geprüft werden. Der Waschvorgang dient auch dazu, Reste des Reduktionsmittels aus Schritt c) zu entfernen. Der Katalysator wird dann bei Temperaturen von höchstens 150°C, vorzugsweise im Stickstoff- oder Luftstrom getrocknet.

Schließlich ist ein Zusatz von mindestens einer Alkalimetallverbindung notwendig. Der Katalysator wird vorzugsweise mit einer wäßrigen Lösung von Kaliumacetat imprägniert und dann getrocknet. Der Gehalt des fertigen Katalysators an Kalium liegt zwischen 1 und 4 Gew.-%, vorzugsweise zwischen 1,5 und 3 Gew.-%.

Die Herstellung des Vinylacetats erfolgt durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol.-% (bezogen auf das essigsäurefreie Gasgemisch). Manchmal ist jedoch auch eine Verdünnung mit inerten Gasen wie Stickstoff oder Kohlendioxid vorteilhaft. Besonders Kohlendioxid eignet sich zur Verdünnung bei einer Kreislauf-Fahrweise, da es in geringen Mengen wahrend der Reaktion gebildet wird.

### Beispiel 1

250 ml Siliziumdioxid-Katalysatorträger (hergestellt von Südchemie) in Form von Kugeln mit einem Durchmesser von 7,3 mm wurden mit 85 ml einer wäßrigen Lösung imprägniert, die 4,6 g Na₂PdCl₄ und 1,4 g NaAuCl₄ enthielt. Die Ausfällung der unlöslichen Metallverbindungen wurde durch Zugabe von 283 ml wäßriger Lösung von 17 g Borax erreicht. Das Gefäß wurde daraufhin sofort mit Hilfe eines Rotationsverdampfers (ohne Vakuum) für 2,5 Stunden bei 5 Umdrehungen pro Minute (UpM) rotiert. Die Reduktion wurde anschließend durch die Zugabe von 7 ml Hydrazinhydrat in 20 ml Wasser und sofortigem Rotieren des Gefäßes bei 5 UpM für 1 Stunde erzielt. Anschließend wurden die so behandelten Träger 16 Stunden stehen gelassen. Daraufhin wurde die Flüssigkeit abgegossen und die behandelten Träger mit destilliertem Wasser gewaschen, um die Chloridionen zu entfernen. Dazu wurde eine Wasserflußrate von 200 ml/Minute über ungefähr 5 Stunden benötigt. Die so erhaltenen Pellets wurden für eine Stunde bei 100°C getrocknet. Der reduzierte Katalysator wurde mit einer wäßrigen Lösung getränkt, die 10 g Kaliumacetat enthielt, und deren Volumen der Absorptionsfähigkeit des trocknen Trägermaterials entsprach. Der Katalysator wurde dann erneut getrocknet.
200 ml des fertigen Katalysators wurden in ein Reaktionsrohr von 20 mm Innendurchmesser und 1,5 m Länge eingefüllt. Dann wurde bei einem Druck von 8 bar am Reaktoreingang und einer Manteltemperatur von 150°C das umzusetzende Gasgemisch über den Katalysator geleitet. Dieses Gasgemisch bestand aus 50 Vol.-% Ethylen, 12 Vol.-% Essigsäure, 6 Vol.-% Sauerstoff und 32 Vol.-% Stickstoff. Die Ergebnisse sind in Tabelle I wiedergegeben.

### Vergleichsbeispiel 1

Es wurde gearbeitet wie in Beispiel 1, außer daß die Ausfällung der unlöslichen Metallverbindungen durch die Zugabe von 283 ml einer wäßrigen Lösung von 2 g NaOH bewirkt wurde. Die Ergebnisse sind in Tabelle I wiedergegeben.

**Tabelle I**

| | Beispiel 1 | Vergleichsbeispiel 1 |
|---|---|---|
| Leistung | 837 | 840 |

| Selektivitäten: | | |
|---|---|---|
| Kohlendioxid | 9,13 | 9,20 |
| Ethylacetat | 0,09 | 0,12 |
| Hochsieder | 0,58 | 1,53 |
| Einheiten: Leistung: Gramm Vinylacetat pro Liter Katalysator pro Stunde Selektivitäten: Molprozent bezogen auf umgesetztes Ethylen | | |

## Patentansprüche

1. Katalysator zur Herstellung von Vinylacetat in der Gasphase aus Ethylen, Essigsäure und Sauerstoff oder Sauerstoff enthaltenden Gasen bei gleichzeitig geringer Hochsiederbildung, der Palladium und/oder dessen Verbindungen, Gold und/oder dessen Verbindungen sowie Alkalimetallverbindungen auf einem teilchenförmigen Träger enthält, dadurch gekennzeichnet, daß der Katalysator zusätzlich Bor oder Borverbindungen enthält.

2. Katalysator nach Anspruch 1, dadurch gekennzeichnet, daß der Borgehalt 0,01 - 1 Gew.-% beträgt.

3. Katalysator nach Anspruch 1, dadurch gekennzeichnet, daß der Borgehalt 0,01 - 0,2 Gew.-% beträgt.

4. Katalysator nach einem der Ansprüche 1 bis 3, dadurch gekennzeichnet, daß er Borate enthält.

5. Katalysator nach einem der Ansprüche 1 bis 4, dadurch gekennzeichnet, daß die Alkalimetallverbindung Kaliumacetat ist.

6. Verfahren zur Herstellung eines Katalysators zur Produktion von Vinylacetat in der Gasphase aus Ethylen, Essigsäure und Sauerstoff oder Sauerstoff enthaltenden Gasen bei gleichzeitig geringer Hochsiederbildung, der Palladium und/oder dessen Verbindungen, Gold und/oder dessen Verbindungen sowie Alkalimetallverbindungen auf einem teilchenförmigen Träger enthält, wobei der Katalysator hergestellt wird indem man
a) den Träger mit löslichen Palladium- und Goldverbindungen imprägniert;
b) die löslichen Palladium- und Goldverbindungen durch eine alkalisch reagierende Lösung auf dem Träger in unlösliche Verbindungen überführt;
c) die unlöslichen Palladium- und Goldverbindungen auf dem Träger durch ein Reduktionsmittel in flüssiger Phase reduziert;
d) den Träger wäscht und anschließend trocknet;
e) den Träger mit einer löslichen Alkalimetallverbindung imprägniert; und
f) den Träger abschließend bei höchstens 150°C trocknet,
dadurch gekennzeichnet, daß vor der abschließenden Trocknung Bor oder Borverbindungen auf den Katalysator aufgebracht werden.

7. Verfahren nach Anspruch 6, dadurch gekennzeichnet, daß der Borgehalt auf dem Katalysator 0,01 - 1 Gew.-% beträgt.

8. Verfahren nach Anspruch 6, dadurch gekennzeichnet, daß der Borgehalt auf dem Katalysator 0,01 - 0,2 Gew.-% beträgt.

9. Verfahren nach einem der Ansprüche 6 bis 8, dadurch gekennzeichnet, daß die in Schritt b) eingesetzte alkalisch reagierende Lösung Borverbindungen enthält.

10. Verfahren nach einem der Ansprüche 6 bis 9, dadurch gekennzeichnet, daß die in Schritt b) eingesetzte alkalische Lösung als Borverbindung Borate enthält.

11. Verfahren nach einem der Ansprüche 6 bis 10, dadurch gekennzeichnet, daß das in Schritt c) eingesetzte Reduktionsmittel in flüssiger Phase eine wäßrige Lösung ist, die Hydrazin oder ein Alkaliborhydrid enthält.

12. Verfahren nach einem der Ansprüche 6 bis 11, dadurch gekennzeichnet, daß das in Schritt c) eingesetzte Reduktionsmittel Natriumborhydrid ist.

13. Verfahren nach einem der Ansprüche 6 bis 12, dadurch gekennzeichnet, daß die in Schritt e) eingesetzte lösliche Alkalimetallverbindung Kaliumacetat ist.

14. Verfahren zur Herstellung von Vinylacetat in der Gasphase aus Ethylen, Essigsäure und Sauerstoff oder Sauerstoff enthaltenden Gasen bei gleichzeitig geringer Hochsiederbildung an einem Katalysator nach einem der Ansprüche 1 bis 5.

## Claims

1. A catalyst for preparing vinyl acetate in the gas phase from ethylene, acetic acid and oxygen or oxygen-containing gases with, at the same time, low high-boiler formation, which catalyst comprises palladium and/or its compounds, gold and/or its compounds and alkali metal compounds on a particulate support, wherein the catalyst further comprises boron or boron compounds.

2. A catalyst as claimed in claim 1, wherein the boron content is 0.01-1% by weight.

3. A catalyst as claimed in claim 1, wherein the boron content is 0.01-0.2% by weight.

4. A catalyst as claimed in any of claims 1 to 3 comprising borates.

5. A catalyst as claimed in any of claims 1 to 4, wherein the alkali metal compound is potassium acetate.

6. A process for preparing a catalyst for producing vinyl acetate in the gas phase from ethylene, acetic acid and oxygen or oxygen-containing gases with, at the same time, low high-boiler formation, which catalyst comprises palladium and/or its compounds, gold and/or its compounds and alkali metal compounds on a particulate support, where the catalyst is prepared by
a) impregnating the support with soluble palladium and gold compounds;
b) converting the soluble palladium and gold compounds on the support into insoluble compounds by means of an alkaline solution;
c) reducing the insoluble palladium and gold compounds on the support by means of a reducing agent in the liquid phase;
d) washing and subsequently drying the support;
e) impregnating the support with a soluble alkali metal compound; and
f) finally drying the support at a maximum of 150°C,
wherein boron or boron compounds are applied to the catalyst prior to the final drying.

7. The process as claimed in claim 6, wherein the boron content on the catalyst is 0.01-1% by weight.

8. The process as claimed in claim 6, wherein the boron content on the catalyst is 0.01-0.2% by weight.

9. The process as claimed in any of claims 6 to 8, wherein the alkaline solution used in step b) comprises boron compounds.

10. The process as claimed in any of claims 6 to 9, wherein the alkaline solution used in step b) comprises borates as boron compound.

11. The process as claimed in any of claims 6 to 10, wherein the reducing agent in the liquid phase used in step c) is an aqueous solution comprising hydrazine or an alkali metal borohydride.

12. The process as claimed in any of claims 6 to 11, wherein the reducing agent used in step c) is sodium borohydride.

13. The process as claimed in any of claims 6 to 12, wherein the soluble alkali metal compound used in step e) is potassium acetate.

14. A process for preparing vinyl acetate in the gas phase from ethylene, acetic acid and oxygen or oxygen-containing gases with, at the same time, low high-boiler formation over a catalyst as claimed in any of claims 1 to 5.

## Revendications

1. Catalyseur pour la préparation d'acétate de vinyle en phase gazeuse à partir d'éthylène, d'acide acétique et d'oxygène ou de gaz contenant de l'oxygène avec une faible formation simultanée de composés de point d'ébullition élevé, qui contient du palladium et/ou des composés de celui-ci, de l'or et/ou des composés de celui-ci ainsi que des composés de métaux alcalins sur un support en forme de particules, caractérisé en ce que le catalyseur contient en outre du bore ou des composés du bore.

2. Catalyseur selon la revendication 1, caractérisé en ce que la teneur en bore est de 0,01 - 1% en poids.

3. Catalyseur selon la revendication 1, caractérisé en ce que la teneur en bore est de 0,01 - 0,2% en poids.

4. Catalyseur selon l'une quelconque des revendications 1 à 3, caractérisé en ce qu'il contient du borate.

5. Catalyseur selon l'une quelconque des revendications 1 à 4, caractérisé en ce que le composé de métal alcalin est l'acétate de potassium.

6. Procédé pour la préparation d'un catalyseur pour la production d'acétate de vinyle en phase gazeuse à partir d'éthylène, d'acide acétique et d'oxygène ou de gaz contenant de l'oxygène avec une faible formation simultanée de composés de point d'ébullition élevé, qui contient du palladium et/ou des composés de celui-ci, de l'or et/ou des composés de celui-ci ainsi que des composés de métaux alcalins sur un support en forme de particules, le catalyseur étant préparé en ce que
a) on imprègne le support avec des composés solubles de palladium et d'or;
b) on transforme les composés solubles de palladium et d'or en composés insolubles par l'intermédiaire d'une solution réagissant de manière alcaline sur le support;
c) on réduit les composés insolubles de palladium et d'or sur le support par un agent réducteur en phase liquide.
d) on lave le support et on le sèche ensuite;
e) on imprègne le support avec un composé soluble de métal alcalin; et
f) on sèche finalement le support à au plus 150°C,
caractérisé en ce que l'on applique sur le catalyseur du bore ou des composés du bore avant le séchage final.

7. Procédé selon la revendication 6, caractérisé en ce que la teneur en bore sur le catalyseur est de 0,01 - 1% en poids.

8. Procédé selon la revendication 6, caractérisé en ce que la teneur en bore sur le catalyseur est de 0,01 - 0,2% en poids.

9. Procédé selon l'une quelconque des revendication 6 à 8, caractérisé en ce que la solution réagissant de manière alcaline utilisée dans l'étape b) contient des composés du bore.

10. Procédé selon l'une quelconque des revendication 6 à 9, caractérisé en ce que la solution alcaline utilisée dans l'étape b) contient des borates comme composé du bore.

11. Procédé selon l'une quelconque des revendications 6 à 10, caractérisé en ce que l'agent réducteur utilisé dans l'étape c) en phase liquide est une solution aqueuse qui contient de l'hydrazine ou un borohydrure d'alcali.

12. Procédé selon l'une quelconque des revendications 6 à 11, caractérisé en ce que l'agent réducteur utilisé dans l'étape c) est le borohydrure de sodium.

13. Procédé selon l'une quelconque des revendications 6 à 12, caractérisé en ce que le composé de métal alcalin soluble utilisé dans l'étape e) est l'acétate de potassium.

14. Procédé pour la préparation d'acétate de vinyle en phase gazeuse à partir d'éthylène, d'acide acétique et d'oxygène ou de gaz contenant de l'oxygène avec une faible formation simultanée de composés de point d'ébullition élevé sur un catalyseur selon l'une quelconque des revendications 1 à 5.
